# EUROPEAN PATENT SPECIFICATION

(11) **EP 3 504 221 B1**
(45) Date of publication and mention of the grant of the patent: **26.11.2025**
(21) Application number: 17843070.8
(22) Date of filing: 14.08.2017
(51) Int. Cl.: C07J 5/00, C07J 13/00, C07J 71/00

(54) **NOVEL PROCESS FOR PREPARATION OF CORTICOSTEROIDS**
NEUARTIGES VERFAHREN ZUR HERSTELLUNG VON CORTICOSTEROIDEN
NOUVEAU PROCÉDÉ DE PRÉPARATION DE CORTICOSTÉROÏDES

(30) Priority: 25.08.2016 IN 201611028947
(43) Date of publication of application: 03.07.2019
(73) Proprietor: Coral Drugs Pvt. Ltd., New Delhi 110001 (IN)
(72) Inventor: TRIPATHI, Vinayak, Delhi 110001 (IN); KUMAR, Rajesh, Delhi 110001 (IN); BHUWANIA, Rohit, Delhi 110001 (IN); BHUWANIA, Binay Kumar, Delhi 110001 (IN)
(74) Representative: Wetzel, Fritz
(86) International application number: PCT/IN2017/050347
(87) International publication number: WO 2018/037423

(56) References cited:
- WO-A1-2012/011106
- WO-A1-2016/120891
- WO-A1-2016/120891
- "Encyclopedia of reagents for organic synthesis; Vol. 7 : Sod - Trim", 17 September 2007, WILEY, Chichester, ISBN: 978-0-470-84289-8, article BANKS R. ERIC ET AL: "1-(Chloromethyl)-4-fluoro-1,4-diazoniabicyclo[2.2.2]octane Bis(tetrafluoroborate)", pages: 1 - 7, XP093133715, DOI: 10.1002/047084289X.rc116.pub2

## Description

### FIELD OF THE INVENTION

The present invention provides a novel process for the preparation of pregnadiene derivatives

### BACKGROUND OF THE INVENTION

Corticosteroids also referred to as glucocorticosteroids, glucocorticoids or just steroids influence different body tissues and produce effects on various responsive cells.

Corticosteroids have been found to possess anti-inflammatory and immunosuppressive properties. Several Corticosteroids such as Flunisolide, Fluocinolone acetonide and Fluocinonide are used for treatment of several disorders.

Corticosteroids with significant structural changes and chemical manipulation have been developed. In general, therapeutic corticosteroids have a 21-carbon steroid skeleton; Modifications to this skeleton selectively alter the degree of anti-inflammatory activity and the metabolic consequences and vary the duration of activity and protein-binding affinity of the resultant compound.

Therapeutic corticosteroids are typically classified based on their relative glucocorticoid and mineralocorticoid potency. Such as, acetonides at C-16, C-17 are to improve potency of anti-inflammatory activity of steroids but possess low systematic activity.

Further, halogenations of steroids molecule by 9-halo or 6, 9 dihalo substitutions increase their systematic activity and enhances both glucocorticoid and mineralocorticoid activity of C-16, C- 17 substituted acetals, ketals, acetonides, fused ring compounds. For example, Flunisolide is a topical corticosteroid having 6-fluoro and C-16, C-17 acetonide group.

The process for preparation of Corticosteroids having C-16, C-17 substituted acetals, ketals, acetonides, fused ring compounds and halogens at C-6, C-9 or both positions have been disclosed at different instances. However, most of these synthetic procedures involve 6 halo or 6, 9 dihalo, 16a, 17a diols or both as starting materials, which are compounds difficult to prepare, handle and purify, the use of toxic solvents or long reaction times are ineffective for large scale synthesis.

For instance, US 3,126,375 discloses a process for the preparation of 6 halo corticoids. The known methods for the preparation of pregnadiene 16, 17acetals, ketals involve the starting material as 6 halo or 6, 9 halo having 16a, 17a diols with aldehydes in the presence of catalyst such as copper sulphate or copper sulphate or perchloric acid solvent which are compounds difficult to prepare and purify and unstable and use of these solvents on large scale require specialized equipment.

WO 03/47329 discloses a method for the preparation of 6α-fluorinated corticosteroids. This application is specific to a 21 ester, 17-hydroxy corticosteroids and does not disclose the various chemicals, starting material, products or reactions and modification thereof. Therefore, the process is not repeatable.

WO-A-2016/120891 discloses in examples 5 and 8 the preparation of 16, 17-acetalised glucocorticoid compounds without a 6-F substitution and involving 9, 11-epoxy-intermediates.

Hence, there is a need to prepare novel Corticosteroids halogenated at C-6, C-9 or both positions and C-16, C-17 acetals, ketals, acetonides, fused ring compounds using stable, easily available starting materials which can be easily purified, convenient, having high yields, industrially scalable and which does not involve the use of harmful solvents.

### OBJECT OF THE INVENTION

An object of the present invention 1s to provide a novel process for the preparation of pregnadiene derivatives, their stereoisomer and intermediate thereof using stable, easily available and purifiable starting materials without the use of toxic and harmful solvents.

### BRIEF DESCRIPTION OF THE INVENTION

The present invention discloses a process for the preparation of pregnadiene derivatives of formula I as recited in claim 1.

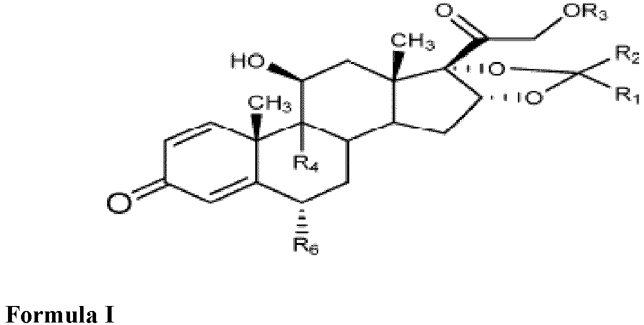

### DETAILED DESCRIPTION OF THE INVENTION

The present invention discloses a novel process for the preparation of pregnadiene derivatives of formula I, their stereoisomer and intermediate thereof. 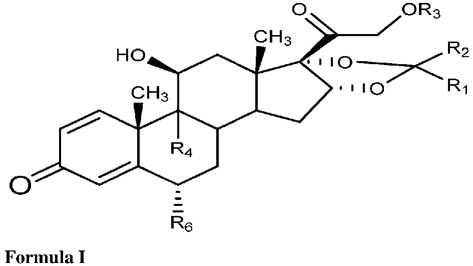 wherein
**R₁** and **R₂** 1s independently hydrogen or Cl -C8 straight, branched alkyl chain saturated or unsaturated cycloalkyl; 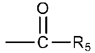
R₃ is hydrogen or ;
R₅ represents Cl-C8 straight, branched alkyl chain or cycloalkyl;
R₄ is independently hydrogen or halogen and R₆ is fluor; comprising the steps of:
   i. epoxidation of compound of formula II to obtain 9,11-oxido derivative of compound of formula III; 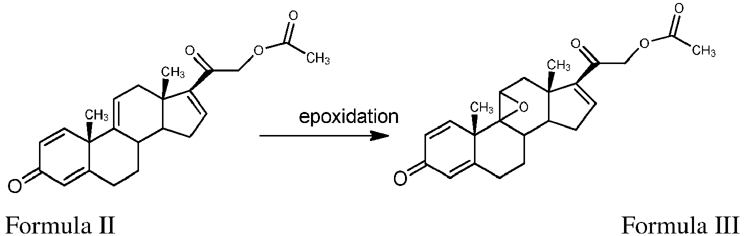
   ii. dihydroxylation and fluorination of compound of formula III to obtain compound of formula IV wherein R6 is F, wherein the fluorination is carried out with a fluorinating agent selected from the group consisting of N-fluoro-benzene sulfonamide, N-fluoro-N chloromethyltrimethylene diamine bistetrafluoroborate, Selectfluor^{®}, 1-fluoropyridinium triflate, 1-fluoropyridinium tetrafluoroborate, or -fluoropyridinium pyridine heptafluorodiborateetc, preferably Selectfluor^{®} and the solvent selected from the group consisting of acetonitrile, isopropenyl acetate, dichloromethane, dimethyl formamide, diethyl ether, and tetrahydrofuran, preferably isopropenylacetate. 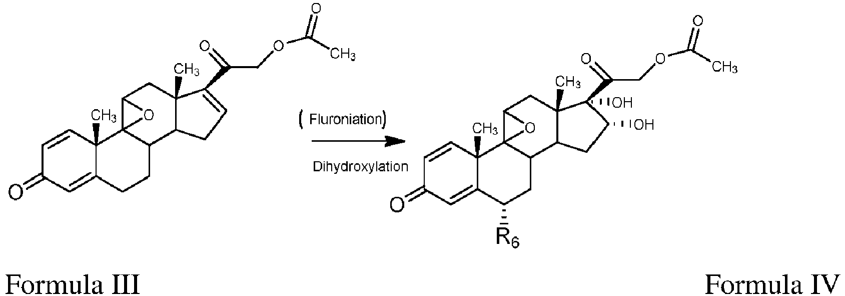
   iii. epoxide ring opening of compound of formula IV followed by acetalisation to obtain compound of formula V and optionally, debromination of compound of formula V, wherein R₄ is Br to obtain compound of formula V when R4 is H, wherein the debromination is carried out presence in of a catalyst selected from the group consisting of chromous or chromium sulfate, chromous or chromium chloride or its hydrate, preferably chromium chloride hexahydrate, a thiol compound of the formula Rt-SH (Formula VIII) wherein Rt is - CH2COOH or -CH2CH2COOH, preferably thioglycolic (thiovanic) acid wherein Rt is -CH2COOH and an aprotic solvent selected from the group consisting of DMF, DMAC, acetone, methylene chloride, THF, acetonitrile, DMSO and mixtures thereof and alcoholic solvents selected from the group selected from methanol, ethanol, isopropanol and butanol, preferably DMF and DMSO. 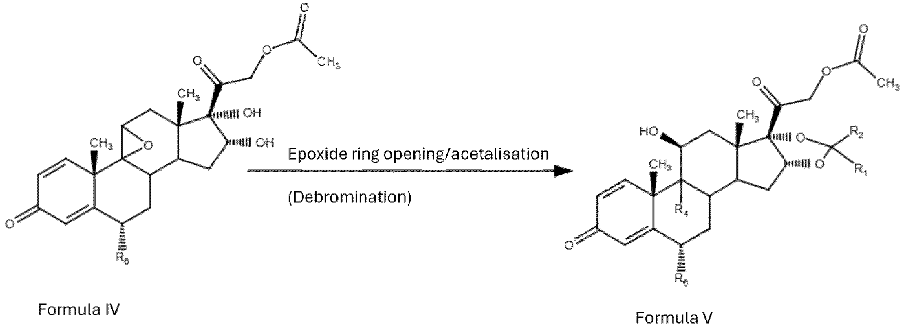
   iv. deacetylation of compound of Formula V to obtain compound of formula I

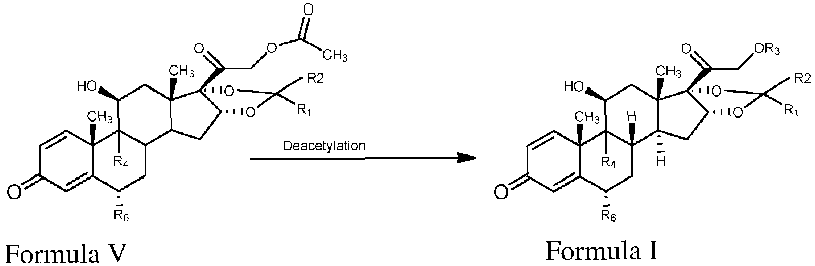

The compounds of formula I or formula V may include but are not limited to the following compounds as presented at Table 1

**Table 1: Exemplary compounds of present invention**

| **Structure** | **Generic name** | **IUPAC Name** |
|---|---|---|
| 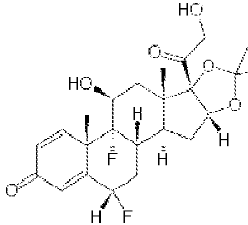 | Fluocinolone acetonide | 1S,2S,4R,8S,9S,11S,12R,13S,19S)-12,19-difluoro- 11-hydroxy-8-(2- hydroxyacetyl)-6,6,9,13-tetramethyl-5,7-dioxapentacyclo[l 0.3.0.0²•⁹.0^{4•3}.0¹³,1 ⁸]icosa-l 4,17-dien-16-one |
| 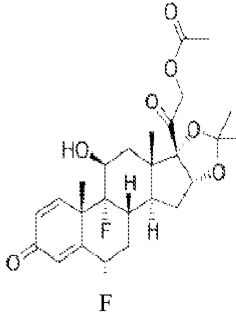 | Fluocinonide | 6a,9-difluoro-l lβ, 16a,17,21-tetrahydroxypregna-1,4-diene-3,20-dione, cyclic 16,17-acetal with acetone,21-acetate |
| 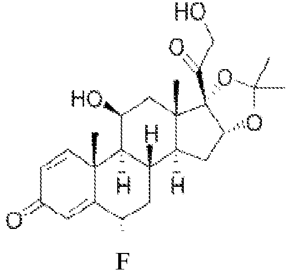 | Flunisolide | 1 S,2S,4R,8S,9S,11S, 12S, *13R,* 19S)-19-fluoro-1 1-hydroxy-8-(2- hydroxyacetyl)-6,6,9,13- tetramethyl-5,7-dioxapentacyclo[l 0.3.0.0²•⁹.0⁴•³.0¹³,1 ⁸]icosa-l 4,17-dien-16-one |

The process of the present invention is explained at scheme 1.

### Scheme 1: Process of the present invention

The process of the present invention comprises of the following steps as described before:
i. epoxidation of compound of formula II to obtain 9,11-oxido derivative of compound of formula III;
ii. dihydroxylation and fluorination of compound of formula III to obtain compound of formula IV wherein **R₆** is F.
iii. epoxide ring opening of compound of formula IV followed by acetalisation to obtain compound of formula V and optionally, debromination of compound of formula V wherein **R₄** is Brto obtain compound of formula V when R4 is **H**
iv. deacetylation of compound of Formula V to obtain compound of formula I

The process of the present invention may be suitably started from the compound of formula II, known by its IUPAC name 2-((10S,13S,14S)-10,13-dimethyl-3-oxo-6,7,8,10,12,13,14,15- octahydro-3H-cyclopenta[a]phenanthren-17-yl)-2-oxoethyl acetate and hereinafter referred to as 3TR (formula II).

3TR is suitably epoxidized by an epoxidizing agent. The epoxidizing agents may be selected from the group consisting of dibromantin, N-bromoacetaminde or N-bromosuccinimide with Perchloric acid to get bromohydrin, followed by alkali treatment from the group consisting of sodium or potassium hydroxide or their carbonates or acetate to form compound of formula (III). The epoxidation of 3TR results in a compound of formula III.

In the present invention, epoxidation of compound of formula (II) is carried out with an epoxidizing agent, which may be selected from the group consisting of dibromantin, N-bromoacetaminde or N- bromosuccinimide with Perchloric acid, preferably dibromantin with perchloric acid in aqueous acetone to get bromohydrin and epoxidation by alkali from the group consisting of sodium or potassium hydroxide or their carbonates or acetate, preferably potassium carbonate to form compound of formula (III).

The process of the present invention involves the fluorination followed by dihydroxylation of the compound of formula III to yield the dihydroxylated fluorinated compound of formula IV. The fluorination of the compound of Formula III is conducted by suitably reacting thecompound of Formula III with a fluorinating agent selected from the group consisting of N- fluoro-benzene sulfonamide, N-fluoro-N-chloromethyltrimethylene diamine bistetrafluoroborate, Selectfluor^{®}, 1-fluoropyridinium triflate, 1-fluoropyridinium tetrafluoroborate, or 1-fluoropyridinium pyridine heptafluorodiborate, preferably Selectfluor^{®} in presence of a solvent and an acid catalyst. The solvent may be selected from the group consisting of acetonitrile, isopropenyl acetate, dichloromethane, dimethyl formamide, diethyl ether, and tetrahydrofuran, preferably isopropenylacetate.

Further, dihydroxylation of the 6-fluoro derivative may be conducted by suitably reacting the compound of Formula III with an oxidizing agent selected from the group consisting of potassium permanganate, potassium dichromate, chromic acid, peroxyacids or mixtures thereof, preferably potassium permanganate, formic acid to form compound of formula (IV). Under acidic conditions formic acid promotes oxidation reaction in the formulated product.

In the present invention, compound of formula (III) is fluorinated with a fluorinating agent selected from the group consisting of N-fluoro-benzene sulfonamide, N-fluoro-N-chloromethyltrimethylene diamine bistetrafluoroborate, Selectfluor^{®}, 1-fluoropyridinium triflate, 1-fluoropyridinium tetrafluoroborate, or 1-fluoropyridinium pyridine heptafluorodiborate etc, preferably Selectfluor^{®} in the presence of a solvent. The solvent is selected from the group consistiong of acetonitrile, isopropenyl acetate, dichloromethane, dimethyl formamide, diethyl ether, and tetrahydrofuran followed by dihydroxylation with an oxidizing agent , preferably selected from the group consisting of potassium permanganate, potassium dichromate, chromic acid, peroxyacids or mixtures thereof, preferably potassium permanganate, formic acid to form compound of formula (IV). Under acidic conditions formic acid promotes oxidation reaction in the formulated product

The epoxide ring opening of compound of formula IV is conducted in the presence of hydrohalic acid followed by acetalisation with aldehyde or ketone to form compound of formula (V). The epoxide ring opening of the compound of Formula IV is conducted by suitably reacting the compound of Formula IV with a hydrohalic acid selected from the group consisting of HF, HCl and HBr, preferably HF.

The acetalisation of the compound of Formula IV is conducted by suitably reacting the compound of Formula IV with an aldehyde or ketone selected from the group consisting offormaldehyde, paraldehyde, acetone, benzaldehyde, acetophenone, diethylketone, cyclohexane corboxaldehyde sodium metabisulphite complex, cyclopentanone preferably acetone.

In the present invention, the epoxide ring opening of compound of formula IV may be conducted in the presence of hydrohalic acid selected from the group consisting of HF, HCl and HBr, preferably HF followed by acetalisation with aldehyde or ketone selected from the group consisting of formaldehyde, paraldehyde, acetone, benzaldehyde, acetophenone, diethylketone, cyclohexane corboxaldehyde sodium metabisulphite complex, cyclopentanone, preferably acetone, to form compound of formula (V).

The compound of formula V is converted to the compound of formula I by deacetylation by reacting with reagents selected from the group consisting of sodium hydroxide and potassium hydroxide and solvent as methanol, methylene chloride, water or their combination thereof.

When carried out, the debromination of compound of formula IV is conducted in presence of a catalyst, a thiol compound and an aprotic solvent to obtain the compound of formula VI when R4 in formula VI is H and this is done under the conditions specified above and in claim 1.

In the present invention, catalyst is selected from the group consisting of chromous or chromium sulfate, chromous or chromium chloride or its hydrate, preferably chromium chloride hexahydrate. The chromium (III) can be recycled to chromium (II) as is known to those skilled in the art. The means for recycling chromium (III) to chromium (II) includes zinc, magnesium, zinc amalgam and magnesium amalgam. Preferred is zinc and magnesium; most preferred is zinc. It is preferred that when the means for recycling is zinc it is present as zinc dust.

Thiols are compounds of the formula Rt-SH (Formula VIII). Rt is -CH2 --COOH or --CH2CH2 --COOH; it is preferred that the thiol be thioglycolic (thiovanic) acid where Rt is --CH2 --COOH. Improved chemical yields result from the use of greater than 1 equivalent of the thiol, preferably from about 1.5 to about 3.0 equivalents.

In the present invention compound of formula (V) is debrominated to form compound of formula (VI) when R4 in formula VI is H in presence of catalyst, Rt---SH (VIII) with an aprotic solvent at appropriate temperature, wherein

Rt---SH Formula VIII

wherein Rt is -CH2COOH or -CH2CH2COOH preferably Rt is -CH2COOH.

Suitable aprotic solvents are DMF, DMAC, acetone, methylene chloride, THF, acetonitrile, DMSO and mixtures thereof. Alcoholic solvents selected from methanol, ethanol, isopropanol and butanol can also be used. Preferred are DMF and DMSO.

In the present invention, aprotic solvent is selected from the group comprising DMF, DMAC, acetone, methylene chloride, THF, acetonitrile, DMSO and mixtures thereof, preferably DMF and DMSO.

The compound of formula IV may undergo epoxide ring opening (not according to the invention) by suitably reacting the compound of Formula IV with a hydrohalic acid selected from the group comprising HF, HCl, and HBr. preferably HF followed by deacetylation to form the compound of Formula A and optionally purified and utilized for commercial purposes. When on C-16 and C-17 of the compound of formula is "OH", then the compound as (11β, 16a)-9-Fluoro-11, 16, 17, 21-tetrahydroxypregna-l, 4-diene-3, 20-dione (Formula A), also known as Triamcinolone.

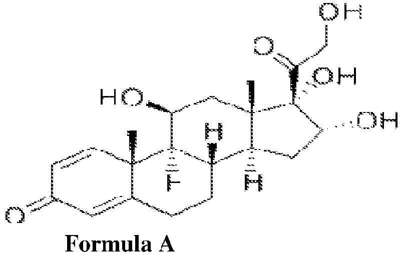

In another embodiment of the present invention, the compound of formula (IV) is converted to compound of formula (**I)** comprising R₁ is -CH₃, R₂ is -CH₃, R3 is -CH₃COO-(acetoxyacetyl), R4 is -F, R₆ is -F by treating with acetone and hydrofluoric acid i.e. Fluocinonide

In yet another embodiment of the present invention, the compound of formula (IV) is converted to compound of formula **(I)** comprising R₁ is -CH₃, R₂ is -CH₃, R₃ is **-H,** , R₄ is -F, R₆ is -F by treating with acetone and hydrofluoric acid followed by deacetylation with Methanol and Methylene chloride in the presence of sodium hydroxide i.e. Fluocinolone acetonide

In yet another embodiment of the present invention, the compound of formula (IV) is converted to compound of formula **(I)** comprising R₁ is -CH₃, R₂ is -CH₃, R₃ is **-H,** R₄ is **-H,** R₆ is -F by treating with acetone and hydrobromic acid followed by sequential step of debromination and deacetylation i.e. Flunisolide.

The compound of formula I may be optionally purified to obtain a pure compound. Such purification may be done by means of crystallization or column chromatography.

### ADVANTAGES OF THE PRESENT INVENTION:

1. The process of the present invention uses commonly available and inexpensive materials.
2. The process is simple and does not involve any toxic materials.
3. The process yields several intermediates that have biological activity and commercial utility.

The invention will now be further illustrated by non limiting examples.

### Working Examples:

### Example-1: Process for preparation of Flunisolide of Formula I from 3TR

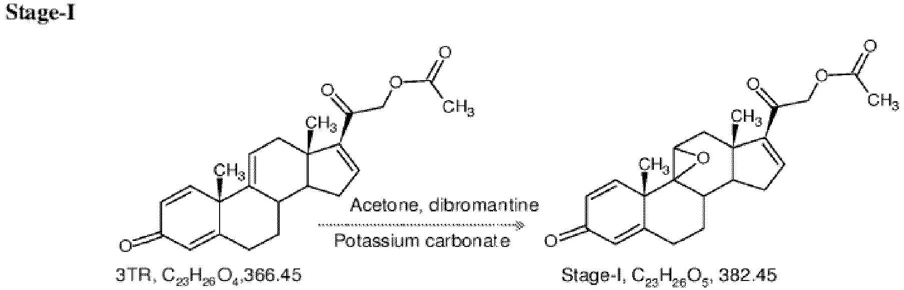

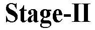

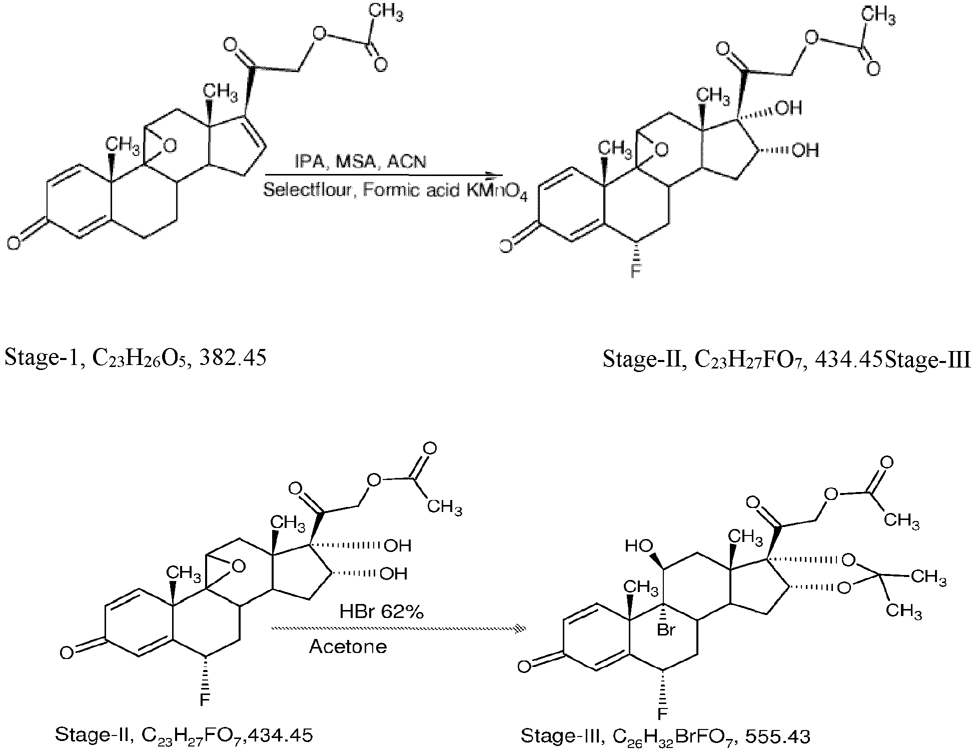

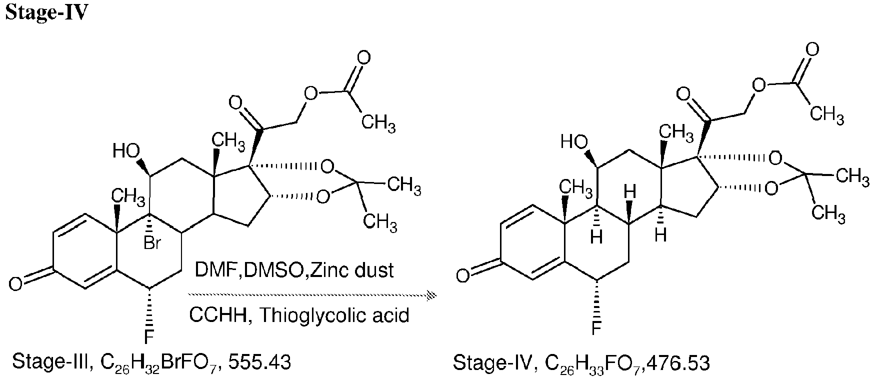

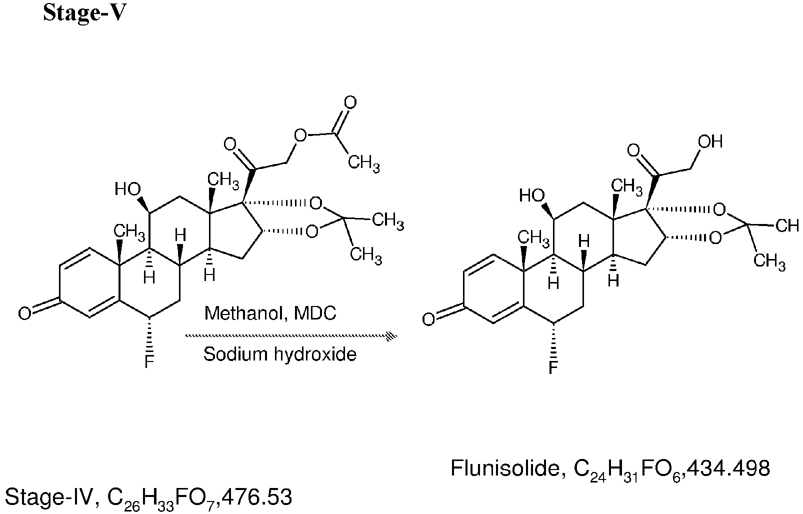

### Scheme 1: Synthesis of Flunisolide from 3TR

### Stage-1: (Epoxidation)

Charge 1.30L of acetone (13.0volume), lO0gm 0f 3TR (0.27mol) in a glass flask, stir till clear solution, cool to -5°C to -10°C, added 4.0ml of perchloric acid solution (0.044mol) in 650ml purified water (6. 5volume) at -5°C to -10°Cand added 50gm of dibromantine (0.18mol) at same temperature. Stir at -5°C to -10°C for 02hours. In-process check by TLC against 3TR, should be absent. Added lO0gm of potassium carbonate solution (0.72mol) in 400ml purified water (4.0volume) at -5°C to 0°C, temperature gradually raised up to 35°c±2°C, stir at same temperature for 12hours. In-process check by TLC against inter-step, should be absent. Cool to 0 C to 5°C, added 36ml of acetic acid (0.63mol) to neutralized the pH of reaction mass, distill the solvent till thick mass under reduced pressure further added 3.0L of purified water (30volume) at 0°C to 5°C, maintaining the temperature 10°C±5°C for 02 hours. Filter and washed with purified water. Dry the wet material at 45°C±2°C until the moisture contents less than 0.50%
Output= 100 gm
Yield= 96%
HPLC Purity=97 .1 %

### Stage-II: (Sequential steps of Dihydroxylation and Fluorination)

Charge 801ml of Isopropenyl acetate (9.0volume), 2.67ml of methane sulphonic acid (0.04mol) in a glass flask. Heated to 85°C±5°C, added 89.0gm of stage-I (0.23mol), stir for 03hours at same temperature. In-process check by TLC against stage-I, should be absent. Cool to 25°C±5°C and adjust pH neutral using 4.0ml of tri ethyl amine (0.03mol). Recovery of isopropenyl acetate under vacuum at below 60°C and degassed with 178ml of acetonitrile (02volume). Charge 979ml of acetonitrile (l l volume), cool to -5°C to -10°C, added 89.0ml of purified water (1.0volume) at same temperature, added 89gm of select fluor (0.25mol) at -5°C to -10°C, stir for 12hours at same temperature. In-process check by TLC against inter step, should be absent. Added 1.l L of purified water (12.4volume) at same temperature, Adjust pH neutral using liq. Ammonia at 15°C±5°C. Charge 445ml of methylene dichloride (5volume) at same temperature, stir, settle, separate methylene chloride layer, distill till last drop and degassed with acetone. Charge 5.0L of acetone (56.18 volume), still till clear solution, cool to - 5°C to -10°C, added 40ml of formic acid (l.06mol) and 60gm of potassium permanganate (0.38mol) at same temperature, stir for one hour. In-process check by TLC against interstep, should be absent, added l0gm of sodium meta bisulphite solution (0.053mol) in 100ml of water (l.0volume) at same temperature, temperature raised up to 15°c and added 30gm of hyflow super cell, further raised the temperature up to 28°C, stir for one hour, filter the reaction mass and washed with l.0L of acetone (l 1.2volume). Collect the filtrate in a glass flask and added l0gm of charcoal activated, stir and filter through hyflow bed and washed with 500ml of acetone (5.6volume), collect the total filtrate in a clean glass flask and recover the acetone under vacuum at below 45°C±5°C till reaction volume app. 400ml. Cool to ambient temperature, added 600ml of purified water (6.7volume), cool to 0°C to 5°C, stir for one hour, filter and washed with purified water, wet material dried at 45°C±5°C till moisture contents less than 1.0%
Output=l00gm
Yield=98.9%
HPLC Purity=81.35%

### Stage-III: (Sequential steps of opening of epoxide ring with hydrohalic acid and acetalisation with aldehyde or ketone)

Charge 25ml of Hydro bromic acid 62% (0.33mol) in a glass flask and cool to -5°C to -10°C, added 5.0gm of stage-11 (0.012mol) at same temperature, stir for one hour. In-process check by TLC against stage-II, should be absent. Added 5.0ml of acetone (1.0volume) at -10°C±2°C, stir for one hour. In-process check by TLC against inter step, should be absent. Quenched the reaction mass in 100ml of chilled purified water (20volume) at 0°C to 5°C, stir for 03hours, filter and washed with purified water till neutral pH, wet material dried at 40°C±5°C till moisture content less than 3.0%
Output=5.0gm
Yield =78.3%
HPLC Purity=88.74%

### Stage-IV: (Debromination)

Charge 4.1ml of N, N-dimethyl form amide (0.82volume) in a glass flask under argon gas bubbling, added 3.0ml of N, N-dimethyl sulphoxide (0.60volume), 0.5gm of chromium chloride hexahydrate (0.002mol), 0.58gm of zinc dust (0.009mol) at ambient temperature. Cool to -7°C to-10°C, added 1.91ml of thioglycolic acid (0.027mol) at same temperature and added 5gm of stage-111 (0.009mol) solution in 25ml of N, N-dimethyl form amide (5volume) at same temperature, stir for 02hours. In-process check by TLC against stage-III, should be absent. Reaction mass quenched in 200ml of purified water (40volume), stir for one hour at 10°C±2°C, filtered and washed with purified water, wet material dried at 40°C±5°C till moisture content less than 3.0%
Output=3.6gm
Yield=83.9%
HPLC Purity= 80.5%

### Stage-V: (Deacetylation)

Charge 30ml of methanol (10volume), 30ml of methylene chloride (10volume) in a glass flask under argon gas bubbling, added 3.0gm of stage-IV (0.0063mol), cool to -5°C±2°C, added 0.06gm of sodium hydroxide (0.0015mol) solution in 3.0ml of methanol (1.0volume) at - 5°C±2°C, stir for 02hours at same temperature. In-process check by TLC against stage-III, should be absent. Adjust pH neutral using 0.09ml of acetic acid (0.0016mol), distilled the methanol and methylene chloride mixture under vacuum at below 40°C till thick mass, cool to ambient temperature and added 9.0ml of purified water (03volume), cool to 0°C to 5°C and stir for one hour, filter and washed with purified water, wet material dried at 45°C±5°C till loss on drying less than 1.0%
Output=2.40gm
Yield=87.60%
HPLC Purity= 98.4
Specific rotation: +104.78°

### Example - 2: Process for preparation of Fluocinolone acetonide of Formula I from 3TR

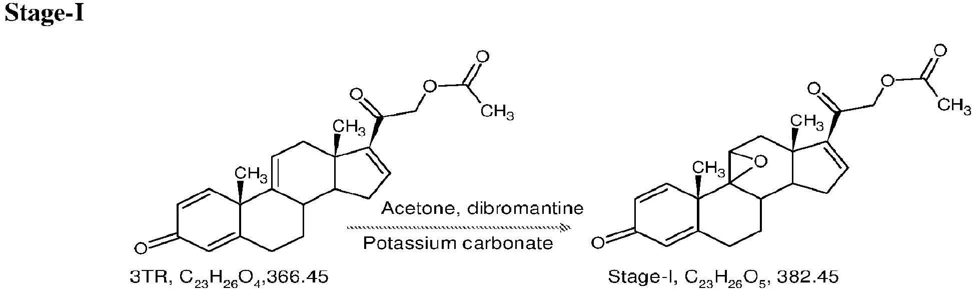

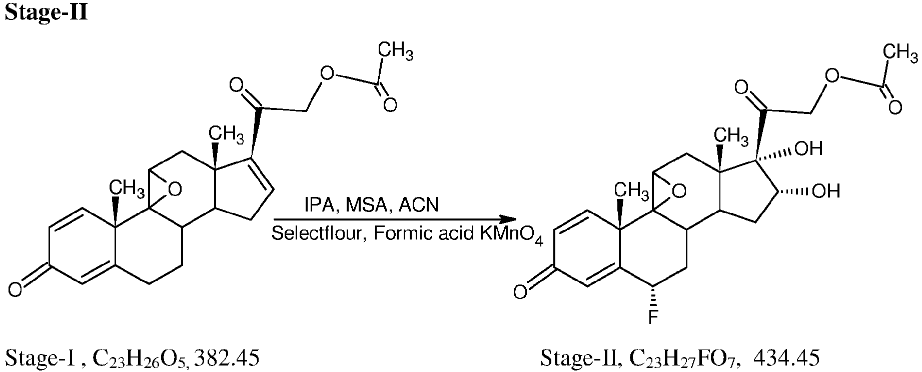

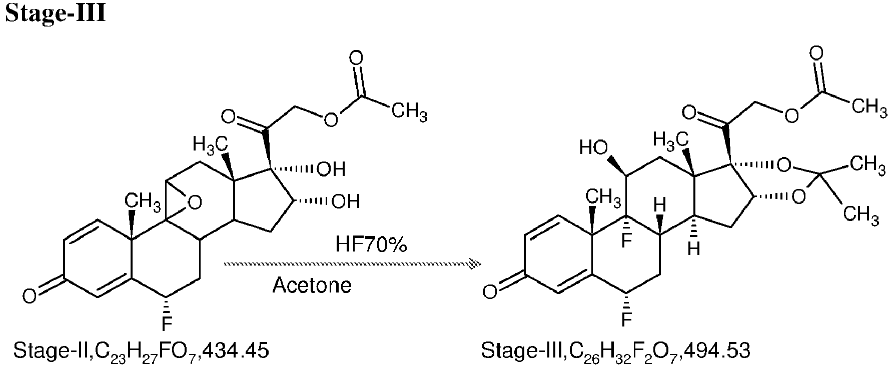

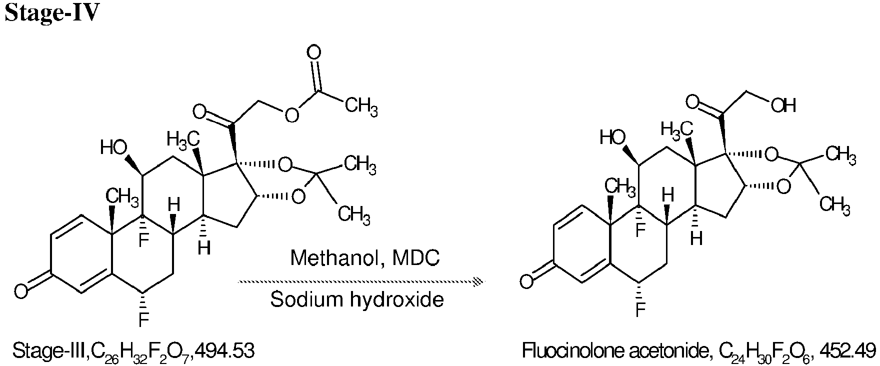

### Scheme 2: Synthesis of Fluocinolone acetonide from 3TR

### (Epoxidation)

Charge 130ml of acetone (13.0volume), 10gm 0f 3TR (0.027mol) in a glass flask, stir till
clear solution, cool to -5°C to -10°C, added 0.4ml of perchloric acid (0.0044mol) solution in 65ml of purified water (6.5volume) at -5°C to -10°C and added 5.0gm of dibromantine (0.018mol) at same temperature. Stir at -5°C to -10°C for 02hours. In-process check by TLC against 3TR, should be absent. Added 10gm of potassium carbonate (0.072mol) solution in 40ml of purified water (4.0vlume) at -5°C to 0°C, temperature gradually raised up to 35°C±2°C, stir at same temperature for 12hours. In-process check by TLC against inter-step, should be absent. Cool to 0°C to 5°C, added 3.6ml of acetic acid (0.063mol) to neutralized the pH of reaction mass, distill the solvent till thick mass under reduced pressure further added 0.30L of purified water(30volume) at 0°C to 5°C, maintaining the temperature 10°C±5°C for 02hours. Filter and washed with purified water. Dry the wet material at 45°C±2°C until the moisture contents less than 0.50%
Output=10 gm
Yield= 96 %
HPLC Purity=97.0%

### Stage-II: (Sequential steps of Fluorination and Dihydroxylation)

Charge 81ml of Isopropenyl acetate (9.0volume), 0.27ml of methane sulphonic acid (0.004mol) in a glass flask. Heated to 85°C±5°C, added 9.0gm of stage-I (0.024mol), stir for 03hours at same temperature. In-process check by TLC against stage-I, should be absent. Cool to 25°C±5°C and adjust pH neutral using 0.40ml of triethylamine (0.0029 mol). Recovery of isopropenyl acetate under vacuum at below 60°C and degassed with 18ml of acetonitrile (02volume). Charge 100ml of acetonitrile (11.1 volume), cool to -5°C to -10°C, added 9.0ml of purified water (l.0volume) at same temperature, added 9gm of selectfluor (0.025mol) at -5°C to -10°C, stir for 12 hours at same temperature. In-process check by TLC against inter step, should be absent. Added 110ml of purified water (12.2volume) at same temperature, Adjust pH neutral using liq. Ammonia at 15°C±5°C. Charge 45ml of methylene dichloride (5volume) at same temperature, stir, settle, separate methylene chloride layer, distill till last drop and degassed with acetone. Charge 500ml of acetone (56.18volume), still till clear solution, cool to -5° C to -10°C, added 4.0ml of formic acid (l.06mol) and 6.0gm of potassium permanganate (0.38mol) at same temperature, stir for one hour. In-process check by TLC against inter step, should be absent, added 1.0gm of sodium metabisulphite (0.0053mol) solution in 10ml of water at same temperature, temperature raised up to 15°C and added 3.0gm of hyflow super cell, further raised the temperature up to 28°C, stir for one hour, filter the reaction mass and washed with 100ml of acetone (11.lvolume). Collect the filtrate in a glass flask and added 1.0gm of charcoal activated, stir and filter through hyflow bed and washed with 50ml of acetone (5.6volume), collect the total filtrate in a clean glass flask and recover the acetone under vacuum at below 45°C±5°C till reaction volume app. 40ml. Cool to ambient temperature, added 60ml of purified water (6.7volume), cool to 0°C to 5°C, stir for one hour, filter and washed with purified water, wet material dried at 45°C±5°C till moisture contents less 1.0%
Output=9.2gm
Yield=80.96%
HPLC Purity=81.0%

### Stage-III: (Sequential steps of opening of epoxide ring with hydrohalic acid and acetalisation with aldehyde or ketone)

Charge 36.8ml of Hydrofluoric acid 70% (1.52mol) in a HDPE reactor and cool to -25°C to - 30°C, added 9.2gm of stage-11 (0.021mol) at -25°C to -30°C, stir for 04hours. In-process check by TLC against stage-11, should be absent. Cool to -45°C to -50°C and added 5.52ml of acetone (0.076mol) at same temperature, stir for 02hours. In-process check by TLC against inter step, should be absent. Added 18.4ml of purified water (02volume) up to 0°C adjust pH neutral using 147.2ml of liq. Ammonia (16.0volume) at 0°C to 5°C stir and filter and washed with purified water, wet material dried at 45°C to 50°C till moisture content less than 1.0%
Output=8.20gm
Yield=78.32%
HPLC Purity=80.0%

### Purification:

Charge 80ml of methylene chloride (9.8volume), 80ml of methanol (9.8volume) in a glass flask, added 8.0gm of stage-111 (0.016mol) and stir till clear solution, added 0.8gm of charcoal activated, stir for 30min.,filter through hyflow bed and washed with 8.0ml of methanol and 8.0ml of methylene chloride mixture, collect the filtrate in a glass flask and distilled to attained the temperature up to 60°C, apply vacuum and distilled till thick mass, cool to 0°C to 5°C, stir for 0ne hour, filter and washed with 8.0ml of chilled methanol, wet material dried at 45°C±5°C till moisture content less than 1.0%
Output=7.20gm
HPLC Purity=87.0%

### Stage-IV: (Deacetylation)

Charge 70ml of methanol (10volume), 70ml of methylene chloride (10volume) in a glass flask under argon gas bubbling, added 7.0gm of stage-III (0.014mol), cool to -5°C±2°C, added 0.14gm of sodium hydroxide (0.0035mol) solution in 7.0ml of methanol at -5°C±2°C, stir for 02hours at same temperature. In-process check by TLC against stage-111, should be absent. Adjust pH neutral using 0.21ml of acetic acid, distilled the methanol and methylene chloride mixture under vacuum at below 40°C till thick mass, cool to ambient temperature and added 10.5ml of purified water (1.5volume), cool to 0°C to 5°C and stir for one hour, filter and washed with purified water, wet material dried at 45°C±5°C till moisture content less than 1.0%.
Output=3.25gm
Yield=50.78% HPLC
Purity=90.0%

### Stage-V: (purification)

Charge 30ml of methylene chloride (10volume), 30ml of methanol (10volume) in a glass flask, added 3.0gm of stage-IV (0.0066mol) and stir till clear solution, added 0.3gm of charcoal activated, stir for 30min.,filter through hyflow bed and washed with 3.0ml of methanol and 3.0ml of methylene chloride mixture, collect the filtrate in a glass flask and distilled to attained the temperature up to 60°C, apply vacuum and distilled till thick mass, cool to 0°C to 5°C, stir for one hour, filter and washed with 3.0ml of chilled methanol, wet material dried at 45°C±5°C till loss on drying less than 1.0%
Output= 1.20gm
HPLC Purity=88.7 %
Specific rotation: + 108.51 °

Further purification required to achieve purity of >98% with desired solvent.

### Example-3 Process for preparation of Fluocinonide of Formula A from 3TR

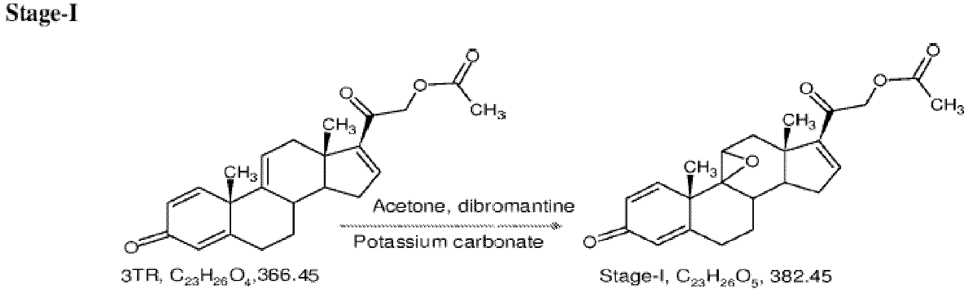

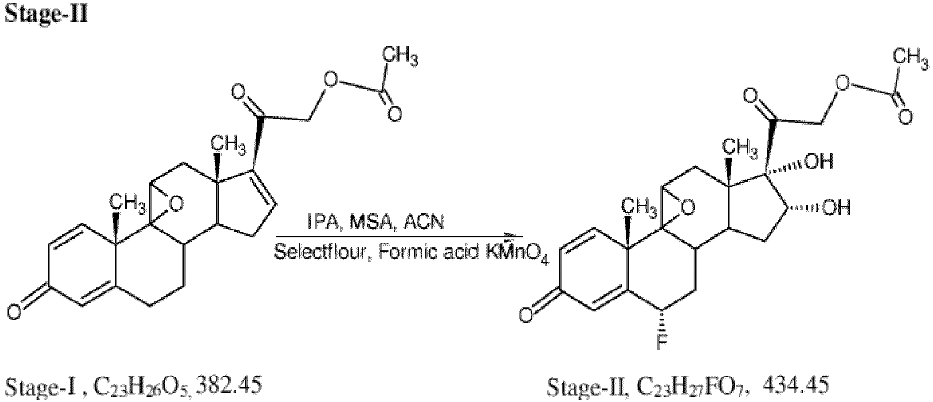

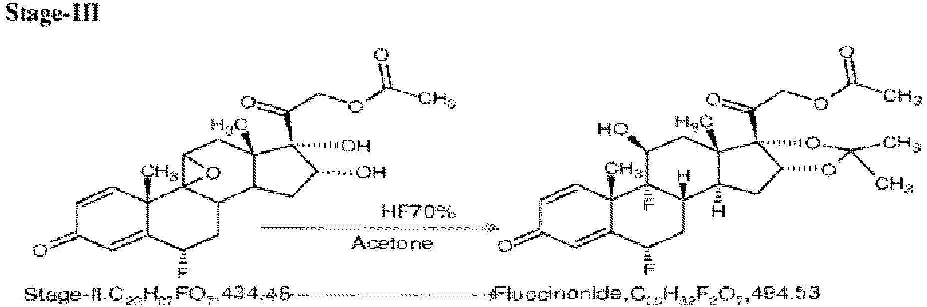

### Scheme 3: Synthesis of Fluocinonide from 3TR

### Stage-1: (Epoxidation)

Charge 130ml of acetone (13.0volume), 10gm 0f 3TR (0.027mol) in a glass flask, stir till clear solution, cool to -5°C to -10°C, added 0.4ml of perchloric acid (0.0044mol) solution in 65ml purified water (6.5volume) at -5°C to -10°C and added 5.0gm of dibromantine (0.018mol) at same temperature. Stir at -5°C to -10°C for 02 hours. In-process check by TLC against 3TR, should be absent. Added 10gm of potassium carbonate (0.072mol) solution in 40ml purified water (4.0volume) at -5°C to 0°C, temperature gradually raised up to 35°C±2°C, stir at same temperature for 12hours. In-process check by TLC against inter-step, should be absent. Cool to 0°C to 5°C, added 3.6ml of acetic acid (0.063mol) to neutralized the pH of reaction mass, distill the solvent till thick mass under reduced pressure further added 0.30L of purified water (30volume) at 0°C to 5°C, maintaining the temperature I0°C±5°C for 02hours. Filter and washed with purified water. Dry the wet material at 45°C±2°C until the moisture contents less than 0.50%
Output=1gm
Yield=96 %
HPLC Purity=97.0%

### Stage-II: ((Sequential steps of Fluorination and Dihydroxylation)

Charge 81ml of isopropyl acetate (9.0volume), 0.27ml of methane sulphonic acid (0.004mol) in a glass flask. Heated to 85°C±5°C, added 9.0gm of stage-1 (0.024mol), stir for 03hours at same temperature. In-process check by TLC against stage-I, should be absent. Cool to 25°C±5°C and adjust pH neutral using 0.40ml of tri ethyl amine (0.003mol). Recovery of isopropenyl acetate under vacuum at below 60°C and degassed with 18ml of acetonitrile (02volume). Charge 100ml of acetonitrile (11.0volume), cool to -5°C to -10°C, added 9.0ml of purified water (1.0volume) at same temperature, added 9gm of selectfluor (0.025mol) at -5°C to -10°C, stir for 12hours at same temperature. In-process check by TLC against inter step, should be absent. Added 110ml of purified water (12.2volume) at same temperature, Adjust pH neutral using liq. Ammonia at 15°C±5°C. Charge 45ml of methylene dichloride (5volume) at same temperature, stir, settle, separate methylene chloride layer, distill till last drop and degassed with acetone. Charge 500ml of acetone (56.18volume), still till clear solution, cool to -5°C to -10°C, added 4.0ml of formic acid (l.06mol) and 6.0gm of potassium permanganate (0.38mol) at same temperature, stir for one hour. In-process check by TLC against inter step, should be absent, added 1.0gm of sodium meta bisulphite (0.0053mol) solution in 10ml of water(1.1volume) at same temperature, temperature raised up to 15°C and added 3.0gm of hyflow super cell, further raised the temperature up to 28°C, stir for one hour, filter the reaction mass and washed with 100ml of acetone (11.1volume). Collect the filtrate in a glass flask and added 1.0gm of charcoal activated, stir and filter through hyflow bed and washed with 50ml of acetone (5.6volume), collect the total filtrate in a clean glass flask and recover the acetone under vacuum at below 45°C±5°C till reaction volume app. 40ml. Cool to ambient temperature, added 60ml of purified water (6.7volume), cool to 0°C to 5°C, stir for one hour, filter and washed with purified water, wet material dried at 45°C±5°C till moisture contents less than 1.0%
Output=8.2gm
Yield=80.24% HPLC
Purity=81%

### Stage-III (Sequential steps of opening of epoxide ring with hydrohalic acid and acetalisation with aldehyde or ketone)

Charge 32.0ml of Hydrofluoric acid 70% (l.32mol) in a HDPE reactor and cool to -25°C to - 30°C, added 8.0gm of stage-11 (0.018mol) at -25°C to -30°C, stir for 04hours. In-process check by TLC against stage-II should be absent. Cool to -45°C to -50°C and added 4.8ml of acetone (0.066mol) at same temperature, stir for 02hours. In-process check by TLC against inter step, should be absent. Added 12.0ml of purified water (l.5volume) up to 0°C adjust pH neutral using 128.0ml of liq. Ammonia (16volume) at 0°C to 5°C stir and filter and washed with purified water, wet material dried at 45°C to 50°C till moisture content less than 1.0%
Output=3.52gm
Yield=38.68%
HPLC Purity=80%

### Purification:

Charge 30ml of methylene chloride (l0volume), 30ml of methanol (l0volume)in a glass flask, added 3.0gm of stage-III (0.006mol) and stir till clear solution, added 0.3gm of charcoal activated, stir for 30min.,filter through hyflow bed and washed with 3.0ml of methanol and 3.0ml of methylene chloride mixture, collect the filtrate in a glass flask and distilled to attained the temperature up to 60°C, apply vacuum and distilled till thick mass, cool to 0°C to 5°C, stir for one hour, filter and washed with 3.0ml of chilled methanol, wet material dried at 45°C±5°C till loss on drying less than 1.0%
Output=1.50gm
HPLC Purity=96%
Specific rotation: +86.96°

## Claims

1. A novel process for the preparation of pregnadiene derivatives of formula I, their stereoisomer and intermediate thereof, 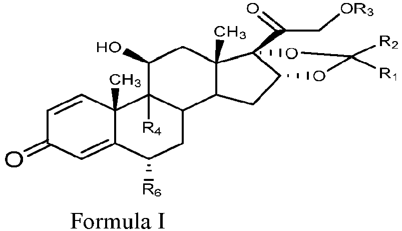 wherein
**R₁** and **R₂** is independently hydrogen or C1-C8 straight, branched alkyl chain, saturated or unsaturated cycloalkyl;
**R₃** is hydrogen or 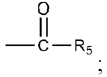
**R₅** represents C1-C8 straight, branched alkyl chain or cycloalkyl;
**R₄** is hydrogen or halogen and **R₆** is fluor;
comprising the steps of:
i. epoxidation of compound of formula II to obtain 9,11-oxido derivative of compound of formula III; 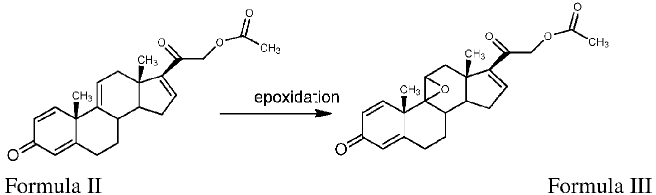
ii. dihydroxylation and fluorination of compound of formula III to obtain compound of formula IV wherein R₆ is F, wherein the fluorination is carried out with fluorinating agent selected from the group consisting of N-fluoro-benzene sulfonamide, N-fluoro-N chloromethyltrimethylene diamine bistetrafluoroborate, Selectfluor^{®}, 1-fluoropyridinium triflate, 1-fluoropyridinium tetrafluoroborate, or -fluoropyridinium pyridine heptafluorodiborateetc, preferably Selectfluor^{®} and the solvent selected from the group comprising acetonitrile, isopropenyl acetate, dichloromethane, dimethyl formamide, diethyl ether, and tetrahydrofuran, preferably isoprenyl acetate; 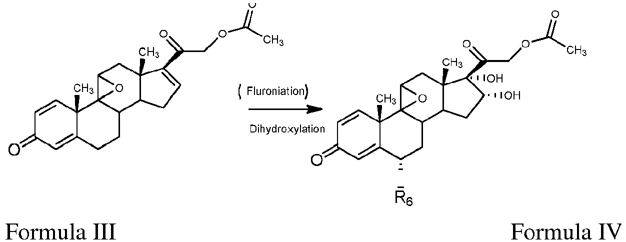
iii. epoxide ring opening of compound of formula IV followed by acetalisation to obtain compound of formula V and optionally debromination of compound of formula V with a proviso R₄ is Br to obtain compound of formula V when R₄ is H, wherein the debromination is carried out presence in of a catalyst selected from the group comprising chromous or chromium sulfate, chromous or chromium chloride or its hydrate, preferably chromium chloride hexahydrate, a thiol compound of the formula Rt-SH (Formula VIII) wherein Rt is - CH2COOH or -CH2CH2COOH, preferably thioglycolic (thiovanic) acid wherein Rt is -CH2COOH and an aprotic solvent selected from the group comprising DMF, DMAC, acetone, methylene chloride, THF, acetonitrile, DMSO and mixtures thereof and alcoholic solvents selected from the group selected from methanol, ethanol, isopropanol and butanol, preferably DMF and DMSO; 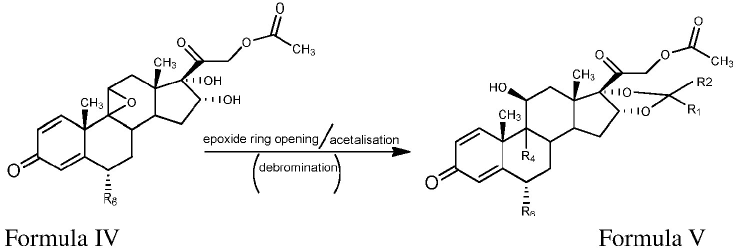
iv. deacetylation of compound of Formula V to obtain compound of Formula I.

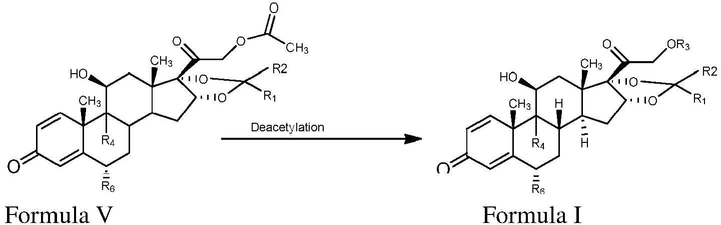

2. The process as claimed in claim 1, wherein the compound of Formula (1) is
i. (1S,2S,4R,8S,9S,11S,12R,13S,19S)-12,19-difluoro-11-hydroxy-8-(2-hydroxyacetyl)-6,6,9,13-tetramethyl-5,7-dioxapentacyclo[10.8.0.02,9.04,8.013,18]icosa-14,17-dien-16-one;
ii. 6α,9-difluoro-11 16 α,17,21-tetrahydroxypregna- l ,4-diene-3,20-dione, cyclic 16,17-acetal with acetone,21-acetate;
iii. 1S,2S,4R,8S,9S,11S,12S,13R,19S)-19-fluoro-11-hydroxy-8-(2-hydroxyacetyl)-6,6,9,13-tetramethyl-5,7-dioxapentacyclo[10.8.0.02,9.04,8.013,18]icosa-14,17-dien-16- one.

3. The process as claimed in claim 1, wherein the epoxidation is carried out with epoxidizing agents selected from the group consisting of dibromantin, N- bromoacetaminde or N-bromosuccinimide with Perchloric acid and an alkali selected from the group consisting of sodium or potassium hydroxide or their carbonates or acetate, preferably potassium carbonate.

4. The process as claimed in claim 1, wherein the dihydroxylation is carried out with an oxidizing agent selected from the group consisting of potassium permanganate, potassium dichromate, chromic acid, peroxyacids or mixtures thereof, preferably potassium permanganate, formic acid.

5. The process as claimed in claim 1, wherein the epoxide ring opening is carried out in the presence of a hydrohalic acid selected from the group consisting of HF, HCl, HBr , preferably HF and acetalisation is carried out with aldehyde or ketone selected from the group consisting of formaldehyde, paraldehyde, acetone, benzaldehyde, acetophenone, diethylketone, cyclohexane, carboxaldehyde, sodium metabisulphite complex, cyclopentanone preferably acetone.

6. The process as claimed in claim 1, wherein the deacetylation is carried out with reagents selected from group consisting of sodium hydroxide, potassium hydroxide and solvent selected from methanol, methylene chloride, water or their combination thereof.

## Patentansprüche

1. Ein neuartiges Verfahren zur Herstellung von Pregnadien-Derivaten der Formel I, deren Stereoisomere und Zwischenprodukte, 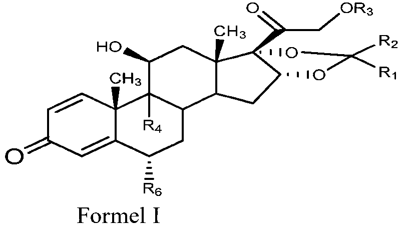 wobei
**R₁** und **R₂** unabhängig voneinander Wasserstoff oder eine geradkettige, verzweigte C1-C8--Alkylkette, gesättigtes oder ungesättigtes Cycloalkyl bedeuten;
**R₃** Wasserstoff oder 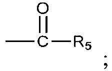
**R₅** eine geradkettige, verzweigte C1-C8-Alkylkette oder Cycloalkyl darstellt;
**R₄** Wasserstoff oder Halogen und **R₆** Fluor ist;
umfassend die folgenden Schritte:
i. Epoxidierung der Verbindung der Formel II, um ein 9,11-Oxido-Derivat der Verbindung der Formel III zu erhalten; 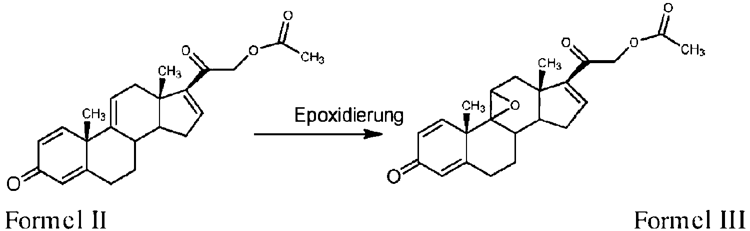
ii. Dihydroxylierung und Fluorierung der Verbindung der Formel III, um eine Verbindung der Formel IV zu erhalten, wobei R₆ F ist, wobei die Fluorierung mit einem Fluorierungsmittel durchgeführt wird, das aus der Gruppe ausgewählt ist, die aus N-Fluorbenzolsulfonamid, N-Fluor-N-Chlormethyltrimethylendiaminbistetrafluorborat, Selectfluor^{®}, 1-Fluorpyridiniumtriflat, 1-Fluorpyridiniumtetrafluorborat oder - Fluorpyridiniumpyridinheptafluordiborat usw., vorzugsweise Selectfluor^{®}, und das Lösungsmittel, ausgewählt aus der Gruppe bestehend aus Acetonitril, Isopropenylacetat, Dichlormethan, Dimethylformamid, Diethylether und Tetrahydrofuran, vorzugsweise Isopropenylacetat, ausgewählt wird; 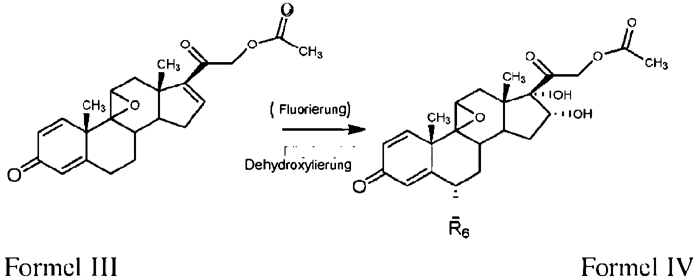
iii. Epoxid-Ringöffnung der Verbindung der Formel IV, gefolgt von einer Acetalisierung, um die Verbindung der Formel V zu erhalten, und gegebenenfalls einer Debromierung der Verbindung der Formel V mit der Maßgabe, dass R₄ Br ist, ,um die Verbindung der Formel V zu erhalten, wenn R₄ **H** ist, wobei die Debromierung in Gegenwart eines Katalysators durchgeführt wird, der aus der Gruppe ausgewählt ist, die Chrom(II)- oder Chromsulfat, Chrom(II)- oder Chromchlorid oder dessen Hydrat, vorzugsweise Chromchloridhexahydrat, einer Thiolverbindung der Formel Rt-SH (Formel VIII), wobei Rt -CH2COOH oder -CH2CH2COOH ist, vorzugsweise Thioglykolsäure (Thiovaninsäure), wobei Rt -CH2COOH ist, und einem aprotischen Lösungsmittel, ausgewählt aus der Gruppe bestehend aus DMF, DMAC, Aceton, Methylenchlorid, THF, Acetonitril, DMSO und Mischungen davon, und alkoholischen Lösungsmitteln, ausgewählt aus der Gruppe, bestehend aus Methanol, Ethanol, Isopropanol und Butanol, vorzugsweise DMF und DMSO, ausgewählt ist; 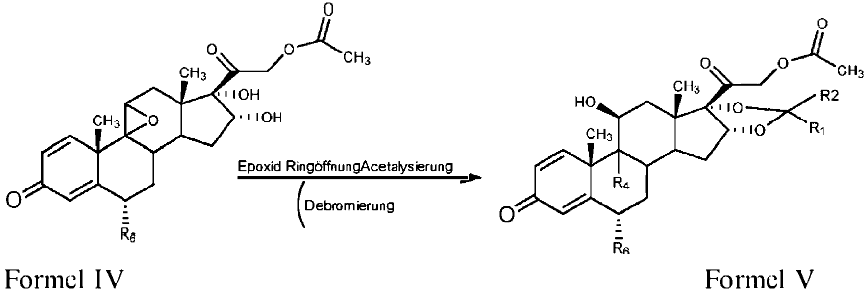
iv. Deacetylierung der Verbindung der Formel V, um die Verbindung der Formel I zu erhalten

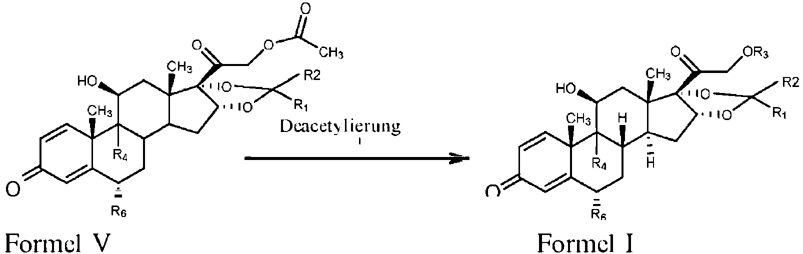

2. Verfahren nach Anspruch 1, wobei die Verbindung der Formel (I)
i. (1S,2S,4R,8S,9S,11S,12R,13S,19S)-12,19-Difluoro-11-hydroxy-8-(2-hydroxyacetyl)-6,6,9,13-tetramethyl-5,7-dioxapentacyclo [10.8.0.02,9.04,8.013, 1 8]icosa-14,17-dien-16-on;
ii. 6α ,9-Difluor-11, 16 α ,17,21-tetrahydroxypregna- 1,4-dien-3,20-dion, cyclisches 1 6,17-Acetal mit Aceton, 21-Acetat;
iii. 1S,2S,4R,8S,9S,11S,12S,13R,19S)- 19-fluoro-11-hydroxy-8-(2-hydroxyacetyl)-6,6,9, 13-tetramethyl-5, 7-dioxapentacyclo[10.8.0.02,9.04,8.013,18]icosa-14,17-dien-16- on.

3. Verfahren nach Anspruch 1, wobei die Epoxidierung mit Epoxidierungsmitteln durchgeführt wird, die aus der Gruppe ausgewählt sind, die aus Dibromantin, N-Bromacetamind oder N-Bromsuccinimid mit Perchlorsäure und einem Alkali besteht, das aus der Gruppe ausgewählt ist, die aus Natrium- oder Kaliumhydroxid oder deren Carbonaten oder Acetaten, vorzugsweise Kaliumcarbonat, besteht.

4. Verfahren nach Anspruch 1, wobei die Dihydroxylierung mit einem Oxidationsmittel durchgeführt wird, das aus der Gruppe ausgewählt ist, die aus Kaliumpermanganat, Kaliumdichromat, Chromsäure, Peroxysäuren oder Mischungen davon besteht, vorzugsweise Kaliumpermanganat, Ameisensäure.

5. Verfahren nach Anspruch 1, wobei die Epoxidringöffnung in Gegenwart einer Halogenwasserstoffsäure, ausgewählt aus der Gruppe bestehend aus HF, HCl, HBr, vorzugsweise HF, durchgeführt wird und die Acetalisierung mit einem Aldehyd oder Keton durchgeführt wird, das aus der Gruppe ausgewählt ist, die aus Formaldehyd, Paraldehyd, Aceton, Benzaldehyd, Acetophenon, Diethylketon, Cyclohexan, Carboxaldehyd, Natriummetabisulfitkomplex, Cyclopentanon, vorzugsweise Aceton, besteht.

6. Verfahren nach Anspruch 1, wobei die Deacetylierung mit Reagenzien durchgeführt wird, die aus einer Gruppe ausgewählt sind, die aus Natriumhydroxid, Kaliumhydroxid besteht, und mit einem Lösungsmittel, das aus Methanol, Methylenchlorid, Wasser oder einer Kombination davon ausgewählt ist.

## Revendications

1. Procédé nouveau pour la préparation de dérivés de prégnadène de formule I, leur stéréoisomère et leur intermédiaire, 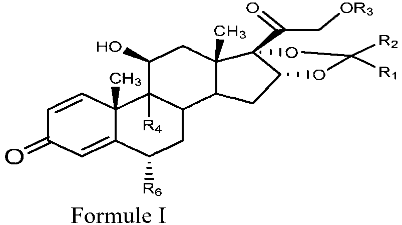 dans laquelle
**R₁** et **R₂** représentent indépendamment l'un de l'autre un atome d'hydrogène ou un groupe alkyle en C1 à C8 à chaîne droite ou ramifiée, un groupe cycloalkyle saturé ou insaturé; 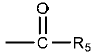
**R₃** est un atome hydrogène, ou
**R₅** représente une chaîne alkyle linéaire ou ramifiée en C1-C8 ou un cycloalkyle ;
**R₄** est un atome d'hydrogène ou un halogène et **R₆** est un atome de fluor ;
comprenant les étapes suivantes:
i. l'époxydation du composé de formule II pour obtenir un dérivé 9,11-oxido du composé de formule III ; 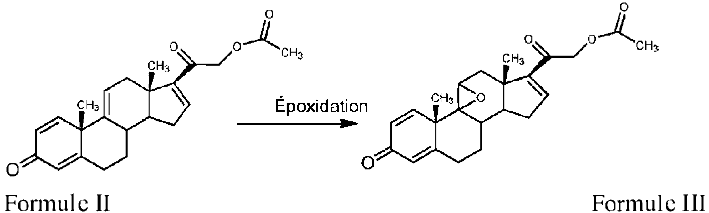
ii. dihydroxylation et fluoration du composé de formule III pour obtenir le composé de formule IV dans laquelle R₆ est F, la fluoration étant réalisée avec un agent de fluoration choisi dans le groupe constitué par le N-fluorobenzènesulfonamide, le N-fluoro-N-chlorométhyltriméthylènediamine bistétrafluoroborate, le Selectfluor^{®}, le triflate de 1-fluoropyridinium, 1-fluoropyridinium tétrafluoroborate ou -fluoropyridinium pyridine heptafluoroborate, de préférence Selectfluor^{®}, et le solvant choisi dans le groupe comprenant l'acétonitrile, l'acétate d'isopropényle, le dichlorométhane, le diméthylformamide, l'éther diéthylique et le tétrahydrofurane, de préférence l'acétate d'isopropényle; 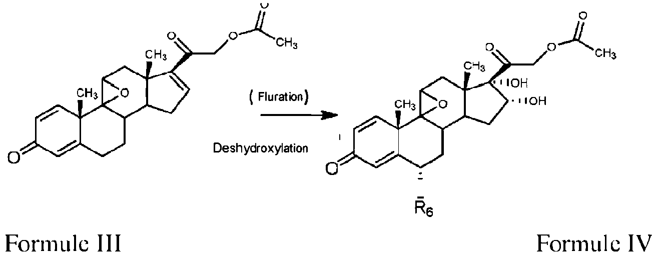
iii. ouverture du cycle époxyde du composé de formule IV suivie d'une acétalisation pour obtenir le composé de formule V et, éventuellement, une débromation du composé de formule V avec une condition R₄ est Br pour obtenir le composé de formule V lorsque R₄ est **H,** dans laquelle la débromation est effectuée en présence d'un catalyseur choisi dans le groupe comprenant le sulfate de chrome ou de chrome, du chlorure de chrome ou de son hydrate, de préférence du chlorure de chrome hexahydraté, un composé thiol de formule Rt-SH (formule VIII) dans laquelle Rt est -CH2COOH ou -CH2CH2COOH, de préférence l'acide thioglycolique (thiovanique) dans lequel Rt est -CH2COOH et un solvant aprotique choisi dans le groupe comprenant le DMF, DMAC, acétone, chlorure de méthylène, THF, acétonitrile, DMSO et leurs mélanges, et des solvants alcooliques choisis dans le groupe constitué par le méthanol, l'éthanol, l'isopropanol et le butanol, de préférence le DMF et le DMSO; 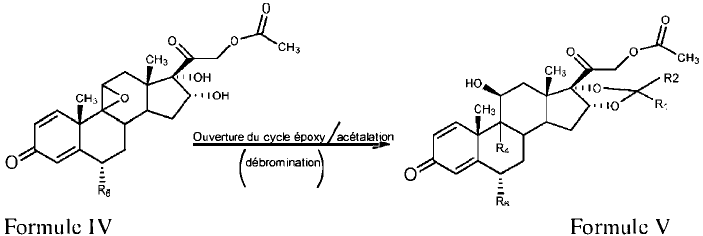
iv. désacétylation du composé de formule V pour obtenir le composé de formule I

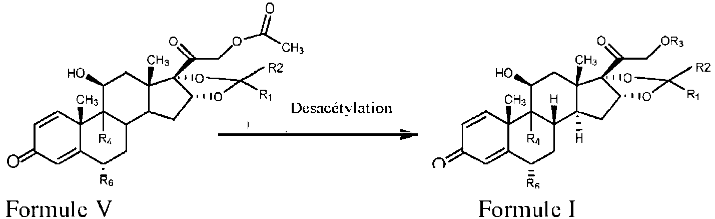

2. Procédé selon la revendication 1, dans lequel le composé de formule (1) est
i. (1S,2S,4R,8S,9S,11S,12R,13S,19S)-12,19-difluoro-11-hydroxy-8-(2-hydroxyacétyl)-6,6,9, 13-tétraméthyl-5,7-dioxapentacyclo [10.8.0.02,9.04,8.013, 1 8]icosa-14, 17-dién-16-on;
ii. 6α ,9-difluoro-11, 16α,17,21-tétrahydroxy prégna- 1,4-diène-3,20-dione, 16,17-acétal cyclique avec l'acétone, 21-acétate;
iii. 1S,2S,4R,8S,9S,11S,12S,13R,19S)- 19-fluoro-11-hydroxy-8-(2-hydroxyacétyl)-6,6,9, 13-tétraméthyl-5,7-dioxapentacyclo[10.8.0.02,9.04,8.013,18]icosa-14,17-dien-16-on.

3. Procédé selon la revendication 1, dans lequel l'époxydation est réalisée avec des agents d'époxydation choisis dans le groupe constitué par la dibromantine, la N-bromoacétamide ou le N-bromosuccinimide avec de l'acide perchlorique et un alcali choisi dans le groupe constitué par l'hydroxyde de sodium ou de potassium ou leurs carbonates ou acétates, de préférence le carbonate de potassium.

4. Procédé selon la revendication 1, dans lequel la dihydroxylation est réalisée avec un agent oxydant choisi dans le groupe constitué par le permanganate de potassium, le dichromate de potassium, l'acide chromique, les peroxyacides ou leurs mélanges, de préférence le permanganate de potassium, l'acide formique.

5. Procédé selon la revendication 1, dans lequel l'ouverture du cycle époxyde est réalisée en présence d'un acide halogénhydrique choisi dans le groupe constitué par HF, HCl, HBr, de préférence HF, et l'acétalisation est effectuée avec un aldéhyde ou une cétone choisi dans le groupe constitué par le formaldéhyde, le paraldéhyde, l'acétone, le benzaldéhyde, l'acétophénone, la diéthylcétone, le cyclohexane, le carboxaldéhyde, le complexe de métabisulfite de sodium, la cyclopentanone, de préférence l'acétone.

6. Procédé selon la revendication 1, dans lequel la désacétylation est réalisée avec des réactifs choisis dans le groupe constitué par l'hydroxyde de sodium, l'hydroxyde de potassium et un solvant choisi parmi le méthanol, le chlorure de méthylène, l'eau ou une combinaison de ceux-ci.
